(19) Europäisches Patentamt
European Patent Office
Office européen des brevets

(11) Publication number: **0 420 683 A2**

# (12) EUROPEAN PATENT APPLICATION

(21) Application number: **90310663.1**

(51) Int. Cl.5: **G01N 29/00**, G01N 33/15

(22) Date of filing: **28.09.90**

(30) Priority: **29.09.89 JP 251858/89**

(43) Date of publication of application:
**03.04.91 Bulletin 91/14**

(84) Designated Contracting States:
**CH DE FR GB IT LI NL SE**

(71) Applicant: **SOGO PHARMACEUTICAL COMPANY LIMITED**
**6-2 Otemachi 2-chome**
**Chiyoda-ku Tokyo(JP)**

(72) Inventor: **Okahata, Yoshio**
**2-11 Nijigaoka 1-chome, Asou-ku**
**Kawasaki-shi, Kanagawa-ken(JP)**
Inventor: **Yomemori, Kazuyuki, c/o Sogo Pharma. Co. Ltd.**
**6-2 Otemachi 2-chome**
**Chiyoda-ku, Tokyo(JP)**
Inventor: **Fujita, Shinsuke, c/o Sogo Pharma. Co. Ltd.**
**6-2 Otemachi 2-chome**
**Chiyoda-ku, Tokyo(JP)**

(74) Representative: **Paget, Hugh Charles Edward et al**
**MEWBURN ELLIS 2 Cursitor Street**
**London EC4A 1BQ(GB)**

## (54) Method of evaluating the physiological activities and structural characteristics of medicinal substances.

(57) A method of evaluating the physiological activities and structural characteristics of medicinal substances, which method comprises determining correlations of (A) at least one of kinds of lipid films, physiological activity factors of medicinal substances and structural characteristics of medicinal substances with (B) at least one of amounts of change in frequency, adsorbed amounts or interacted amounts due to adsorption or interaction of the medicinal substances onto the lipid films and lipid film-water partition coefficients, by use of crystal oscillators cast various lipid films respectively, wherein the lipid film-water partition coefficient is used as a parameter in the structural activity correlation technique for medicinal substances.

EP 0 420 683 A2

# METHOD OF EVALUATING THE PHYSIOLOGICAL ACTIVITIES AND STRUCTURAL CHARACTERISTICS OF MEDICINAL SUBSTANCES

The present invention relates to a method of evaluating the physiological activities and structural characteristics of medicinal substances, and more particularly to a method of evaluating the physiological activities and structural characteristics of medicinal substances such as antibiotics, antimicrobial medicinal substances, local anesthetics, agricultural chemicals, chemicals and the like.

Japanese Patent Application Laid-Open No. 222248/88, for example, discloses a method of detecting bitter or odorous substances by use of a crystal oscillator on the electrode of which an immobilized bilayer film is cast.

From the standpoints of reducing the screenings, animal experiments, etc., which take a long period of time and need high costs, in the research and development of medicines and agricultural chemicals, recently the structural activity correlation technique has been practiced as a technique to numerically evaluate the results from the rule of thumb and know-how for patterning many cases. The structural activity correlation is derived from pharmacological and biochemical hypotheses as well as physicochemical hypotheses, and particularly is based on the physicochemical hypotheses as evident from the fact that the variables of the derived calculation formulas are hydrophobic parameter, i.e. octanol-water partition coefficient, stereo parameter, electron parameter, etc. The results of the practice of the structural activity correlation technique have shown that the hydrophobic parameter may be an effective element for the pharmacological activity in a system in which permeation from the skin or the surface of the body of the insect or plant into the interior of the body becomes necessary. The stereo parameter, electron parameter, etc. are added to the above parameter to effect the linearization of the pharmacological activity according to the calculation formulas comprising 5-7 equations.

The octanol-water partition coefficient used as the hydrophobic parameter in the conventional structural activity correlation technique simply reflects the hydrophobic property only. However, adsorption onto the lipid film or pharmacological activity is not interpretable only by the simple hydrophobic property, and it is necessary for the above interpretation to reflect the size and configuration of the molecule.

It is an object of the present invention to provide a method of evaluating the physiological activities and structural characteristics of medicinal substances, which is capable of remarkably improving the conventional structural activity correlation technique.

It is another object of the present invention to provide a method of evaluating the physiological activities and structural characteristics of medicinal substances, which is capable of quantitatively determining easiness in adsorption of various medicinal substances onto the lipid films and providing teachings of selective easiness in adsorption of molecules of various medicinal substances onto various lipid films.

That is, the present invention provides a method of evaluating the physiological activities and structural characteristics of medicinal substances, which method comprises determining correlations of (A) at least one of kinds of lipid films, physiological activity factors of medicinal substances and structural characteristics of medicinal substances with (B) at least one of amounts of change in frequency, adsorbed amounts or interacted amounts due to adsorption or interaction of the medicinal substances onto the lipid films and lipid film-water partition coefficients, by use of crystal oscillators cast various lipid films respectively.

The present invention further provides a method of evaluating the physiological activities and structural characteristics of medicinal substances, in which method the lipid film-water partition coefficient determined in the above method is used as a parameter in the structural activity correlation technique for medicinal substances.

## Brief Description of the Drawings:

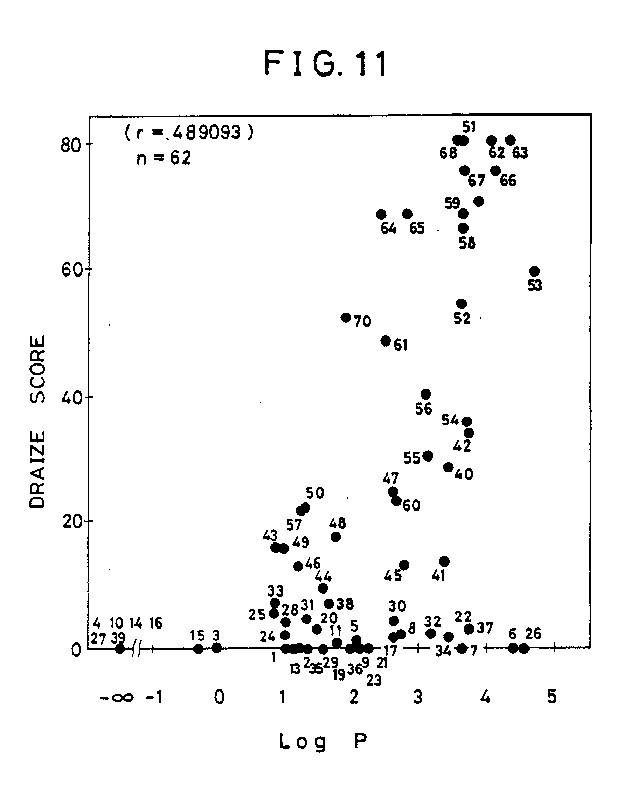

Fig. 1 is a graph showing a frequency change with time, Fig. 2 is a graph showing a correlation between the decreased amount of frequency due to the adsorption of duramycin and the adsorbed amount of duramycin for various lipid films, Fig. 3 is a graph showing correlation of temperature with values of P and D, Fig. 4 is a graph showing a correlation of surface pressure or area per one molecule with partition coefficient (P), Fig. 5 is a graph showing a correlation of $2C_{14}PE$ content or area per one molecule of $2C_{14}PE$ with value of P or D, Fig. 6 is a graph showing a correlation between partition coefficient and antimicrobial activity, Fig. 7 is a graph showing a correlation between octanol-water partition coefficient and antimicrobial activity, Fig. 8 is a graph showing a correlation of the intrinsic potency of various local anesthetics and control substances with partition coefficient, Fig. 9 is a graph showing a correlation between

pH of tetracaine hydrochloride (TC) and partition coefficient, Fig. 10 is a graph showing a correlation between temperature and partition coefficient for tetracaine hydrochloride (TC) in the case where pH is used as a parameter, and Fig. 11 is a graph showing a correlation between draize score and partition coefficient.

## Detailed Description

The crystal oscillator used in the present invention may include any known crystal oscillators, for example, a 9 MHz•AT-cut, gold electrode crystal oscillator, etc.

Measurement of frequency by use of the above crystal oscillator is normally carried out in the distilled water or deionized water. However, when it is necessary for measurement of frequency to be carried out directly in a non-deionized water or an ionic solution, one side barrier covered crystal oscillator, in which one side of both electrodes of the crystal oscillator is covered with a barrier through a predetermined space.

The medicinal substances used in the present invention may include any medicinal substances so long as it is possible to evaluate physiological activities and structural characteristics of the medicinal substances by determining correlations of physiological activities, etc. with lipid film-water partition coefficients, etc., and physiological activities of the medicinal substances are reflected by use of lipid film-water partition coefficient. Examples of the medicinal substances may include antibiotics, antimicrobial medicinal substances, local anesthetics, agricultural chemicals, chemicals and the like.

The present invention makes it possible to provide a method of evaluating the physiological activities and structural characteristics of medicinal substances, which is capable of remarkably improving the conventional structural activity correlation technique in that the lipid film-water partition coefficient in the present invention may be an indication to collectively represent configuration of molecule, polarity of molecule, etc. in addition to hydrophobic properties so as to make it possible to accurately reflect physiological activities of medicinal substances only by the lipid film-water partition coefficient (Log P), and is very effective as a parameter in the practice of the structural activity correlation technique, resulting in making it possible to remarkably improve the conventional structural activity correlation technique for medicinal substances.

The present invention makes it possible to provide a method of evaluating the physiological activities and structural characteristics of medicinal substances, which is capable of quantitatively determining easiness in adsorption of various medicinal substances onto the various lipid films and providing teachings of selective easiness in adsorption of molecules of various medicinal substances onto various lipid films.

The determination of correlations of (A) at least one of kinds of lipid films, physiological activity factors of medicinal substances and structural characteristics of medicinal substances with (B) at least one of amounts of change in frequency, adsorbed amounts or interacted amounts due to adsorption or interaction of the medicinal substances onto the lipid films, and lipid film-water partition coefficients is explained more in detail by the following Examples.

## Example 1

A 9 MHz•AT-cut gold electrode crystal oscillator, on the electrodes of which $2C_{14}$ phosphoethanolamine (PE) based phospholipid Langmuir-Blodgett 30-multibilayer film is cast as a lipid film, was dipped into a 10 mM tris buffer solution having a pH of 7.8 at 60° C, followed by adding duramycin as a cyclic peptide antibiotics to determine frequency changes with time as shown in Fig. 1. As duramycin is adsorbed onto the above film, the frequency slowly decreases, and 20 minutes after, it became a constant value. At the time when shown by a black arrow, the above crystal oscillator was dipped into an aqueous solution identical to the above 10 mM tris buffer solution and free of duramycin with the result that the above value of frequency remained unchanged. The above result shows a strong interaction between the film and duramycin.

Adsorption experiments of duramycin were carried out by use of various lipid LB films having different hydrophilic groups from each other. Amounts of frequency change ($\Delta F$) due to adsorption of duramycin onto other various lipid LB films are shown in Fig. 2 along with those in the above $2C_{14}$PE-LB film. In Fig. 2, $2C_{14}$PE represents $2C_{14}$ phosphoethanolamine based lipid, $2C_{14}$PA represents $2C_{14}$ phosphate based lipid containing phosphate group, $2C_{18}$OH represents $2C_{18}$ alcohol based lipid and galactosyl•diglyceride is an example of lipids having sugar. As evident from Fig. 2, duramycin is hardly adsorbed onto or interacted with the lipid film having sugar or phosphate group, but is specifically adsorbed onto a phosphoethanolamine

based hydrophilic group. Although not shown in Fig. 2, duramycin was hardly adsorbed onto a phosphocholine based lipid film. Since duramycin has three amine groups and carboxylic acid groups respectively, it is considered that an interaction between the above functional groups and the hydrophilic group in the above PE film takes place to be specifically adsorbed onto the PE film.

A penetration coefficient (D) of duramycin into $2C_{14}PE$ film was calculated from an initial frequency change in Fig. 1, and a partition coefficient (P) to the film was calculated from an adsorbed amount obtained from a frequency change at equilibrium adsorption. The values of the penetration coefficient (D) and the partition coefficient (P) were determined as shown in Fig. 3. The values of the penetration coefficient (D) are large when the film is in the crystalline state and showed a minimum near Tc. The value of the partition coefficient (P) showed a maximum near Tc. The interaction between duramycin and $2C_{14}PE$ film may be interpreted by both terms of a disarray and flowability of the film. When the film in a crystalline state at 20-30° C, duramycin is adsorbed onto a defective area on the surface of the film and penetrates into the interior of the film in a little or no amount, but no interaction between duramycin and the PE film takes place, resulting in an increased penetration speed. Near the phase transition temperature Tc, a crystalline phase and a liquid crystals phase coexist, and duramycin is adsorbed in the disarraied area in a large amount, resulting in that the penetration speed is greatly reduced. The film in a uniform liquid crystals state at Tc or higher temperatures shows a slight disarray, resulting in a small adsorbed amount and a moderate penetration speed. As inserted into Fig. 3 and shown, a penetration speed of duramycin into the $2C_{18}OH$-LB film, with which no interaction takes place, is 500 times greater than that into $2C_{14}PE$ film, with which interaction takes place.

Experiments were carried out about effects of a number of the layer of $2C_{14}PE$-LB film. When the film is in the state of liquid crystals at 60° C, a decreased amount of frequency or an adsorbed amount due to adsorption was linearly increased as the number of layer of the LB film was increased to show that duramycin deeply penetrates into the interior of the film to be adsorbed, whereas when the film is in a rigid crystalline state at 20° C, an adsorbed amount was almost independent from the number of the layer of the film to show that an interaction between duramycin and only the surface of the LB film takes place. The above results coincide very well with the results of temperature changes in Fig. 3.

Fig. 4 shows partition coefficient (P) changes of duramycin in the case where a surface pressure was varied on accumulating $2C_{14}PE$ film. The value of the above (P) was linearly increased as the surface pressure for accumulation was decreased or a degree of disarray of the LB film was increased, and coincided with that of a 1:1 ratio adsorption when an area per molecule is 0.55 $nm^2$/molecule. From the fact that an area occupied by one molecule or an area per molecule of $2C_{14}PE$ molecule in the condensed state is 0.4 $nm^2$/molecule and a projected area estimated from a CPK model of duramycin is about 0.6 $nm^2$/molecule, it is considered that the PE film is broadened and disarraied by interaction between duramycin and the PE film. That is, duramycin may easily be adsorbed onto a disarraied film. In the case of Fig. 1 to Fig. 3, the LB film is accumulated by 40 mN/m, and in the case of the film in such a condensed state, one molecule of duramycin may be bonded with 4 to 5 molecules of $2C_{14}PE$ molecule from the results shown in Fig. 5. From a combined consideration with the results of Fig. 3, it is concluded that duramycin narrowly and deeply penetrates into the interior of the film disarraying the film.

In Fig. 5, the values of the above (P) and (D) for duramycin were determined by use of a mixed LB film of $2C_{14}PE$ and $2C_{18}OH$ and are shown in terms of $2C_{14}PE$ content (%). It was demonstrated from the measurement of a $\pi$-A curve that $2C_{14}PE$ and $2C_{18}OH$ were uniformly mixed in the LB film. The value of (P) is first increased as the $2C_{14}PE$ content in the film is decreased when the $2C_{14}PE$ content is about 60% or higher, and remains constant when the $2C_{14}PE$ content is about 6u% or lower to be a (P) value near the (P) value in the case where duramycin bonded with all of the $2C_{14}PE$ molecules at a molar ratio of 1:1 respectively. That is, when the $2C_{14}PE$ molecules were diluted with $2C_{18}OH$ molecules in an amount about equal to the former, in other words, when the area per one molecule of $2C_{14}PE$ was broadened to become 0.55 $nm^2$/molecule, bonding of the $C_{14}PE$ molecule with duramycin at a molar ratio of 1:1 is made possible. This coincides very well with the results obtained by varying the surface pressure for accumulation as shown in Fig. 4.

The results of the above experiments show that correlations of at least one of kinds of lipid films, physiological activity factors of antibiotics as the medicinal substance and structural characteristics of antibiotics as the medicinal substance with at least one of amounts of change in frequency, adsorbed amounts or interacted amounts due to adsorption or interaction of the antibiotics onto the lipid films, and lipid film-water partition coefficients may be determined, and show that the peptide based antibiotics such as duramycin are specifically adsorbed onto a lipid molecule of the cellular film to penetrate into the film, resulting in that the methods shown in the above experiments may be effective means to evaluate physiological activities and structural characteristics of antibiotics as the medicinal substance.

Example 2

It is known that long chain fatty acid and glyceryl esters thereof have antimicrobial activity against Gram positive bacteria, mold, yeast etc., and further that in the case of fatty acid, the antimicrobial activities increase as the chain length is increased in the range of $C_8$ capric acid to $C_{12}$ lauric acid, but decrease or disappear when the chain length is so increased as to be $C_{14}$ myristic acid or higher.

Correlations of antimicrobial activity as the physiological activity factor and structural characteristics of long chain fatty acid as the medicinal substance with adsorbed amounts and partition coefficients were determined by use of a lipid film-coated crystal oscillator as follows.

Adsorbed amounts of various fatty acid in an aqueous phase were determined by using a polyion complex lipid obtained from dialkylammonium salt and polystyrene sulfonic acid as the lipid film in the same crystal oscillator as in Example 1. The lipid film containing bacteria is of a mixture of phospholipid, sugar lipid, cholesterol, etc., but a good correlation between the antimicrobial activity against Streptococcus betahemolytic non-A bacteria and partition coefficient to the lipid film was obtained as shown in Fig. 6, in which the partition coefficient (P) was calculated according to the following equation:

$$\text{Partition Coefficient (P)} = \frac{\text{Concentration of fatty acid in the lipid film}}{\text{Concentration of fatty acid in the aqueous phase}} = \frac{\dfrac{\text{Adsorbed amount of fatty acid}}{\text{Amount of the lipid film}}}{\text{Concentration of fatty acid in the aqueous phase}}$$

The partition coefficient (P) may be a parameter to show that as the value of the partition coefficient (P) is increased, adsorption onto the lipid film becomes easier and, whereas the above value is decreased, adsorption thereonto becomes difficult.

The same correlation as shown in Fig. 6 was obtained for other Gram positive bacteria such as Pheumococci, Streptococcus group A, Candida, S. aureus and the like than the above Streptococcus betahemolytic non-A bacteria to show that the fatty acid is adsorbed onto the lipid film for realizing antimicrobial activity to the Gram positive bacteria as a whole.

For comparison, correlation of the antimicrobial activity with octanol-water partition coefficient (Log $K_{ow}$) representing a parameter of hydrophobic properties of compounds including the above fatty acid was determined as shown in Fig. 7. Although hydrophobic properties simply increases as the chain length is increased, the antimicrobial activity was decreased as the chain length is increased when the chain length becomes longer than $C_{12}$ to show that the antimicrobial activity is decreased when hydrophobic property becomes too strong. That is, in the above adsorption onto the lipid film, in the range where the chain length is so short as to be $C_6$ caproic acid or lower, hydrophilic properties are too strong, resulting in making adsorption difficult, while ones in the range of $C_8$ capric acid to $C_{14}$ myristic acid were adsorbed and showed such properties to deeply penetrate into the interior of the lamella layer of the lipid bilayer film. In the range where the chain length is so long as to be $C_{16}$ palmitic acid or higher, association takes place on the lipid film and adsorption takes place only on the surface of the film, resulting in the adsorption is greatly reduced. That is, the result of the above experiment shows that the antimicrobial activity of fatty acid may be evaluated from the process in which fatty acid is adsorbed onto and penetrates into the interior of the lipid film.

The above experiment by use of the lipid bilayer film-coated crystal oscillator made it possible to evaluate physiological activity of medicinal substances, for example, antimicrobial activity of fatty acid in a readily available manner. It is also considered that the above experiments provide teachings about interaction of the medicinal substances with the lipid film, which was impossible to be understood or interpreted by the conventional manner, for example, octanol-water partition coefficient. In Fig. 6 and Fig. 7, the numbers circled represent chain length of fatty acids, and the antimicrobial activity represents a logarithm of concentrations at a 50% growth inhibition. As the value of Log (concentration at 50% growth inhibition/M) is increased, the antimicrobial activity is decreased, while the above value is decreased, the above activity is increased. According to the graphs in Fig. 6 and Fig. 7, as the value of the axis of ordinates is decreased, the antimicrobial activity is increased.

As evident from this Example, the results of quantitatively determining adsorbed amounts of medicinal

substances onto the lipid film by use of the lipid film-coated crystal oscillator according to the present invention show that the lipid film-water partition coefficient may be an indication to collectively represent configuration of molecule, polarity of molecule, etc. in addition to hydrophobic properties so as to make it possible to accurately reflect physiological activities of medicinal substances only by the lipid film-water partition coefficient (Log P), and is very effective as a parameter in the practice of the structural activity correlation technique, resulting in making it possible to quantitatively determine easiness in adsorption of various medicinal substances onto various lipid films and to provide teachings of selective easiness in adsorption of molecules of various medicinal substances onto various lipid films.

Example 3

Experiments on a mechanism of realization of narcosism were carried out by calculating the partition coefficient of various anesthetics, particularly local anesthetics to the lipid film and comparing with the potency of anesthetics by use of the same crystal oscillator and lipid film as in Example 2.

A lipid bilayer film-coated crystal oscillator was dipped into water, followed by adding respective aqueous solutions of various local anesthetics used in clinical demonstration, for example, dibucaine (DC), tetracaine (TC), bupivacaine (BC), lidocaine (LC), prilocaine (CT), mepivacaine (MC) and procaine (PC) to determine respective partition coefficients from the aqueous phase to the lipid film and compare with the intrinsic potency showing medicinal intensity of the local anesthetics as shown in Fig. 8. The results showed a good linear correlation between the partition coefficient (Log P) and the intrinsic potency, and showed that the anesthetics, which are adsorbed onto the lipid bilayer film more easily, have a higher intrinsic potency. Measurements were made under conditions of 45° C and pH of 7, under which the $2C_{18}N^{+}2C_1/PSS^{-}$ lipid film was in the liquid crystals (Tc = 45° C) and the anesthetics were added in the form of hydrochloride.

It is known that the local anesthetics are solubilized at a lipid moiety of the cellular film of a synapse of the nerve and block ion channels to realize their narcosism, and it is considered that a first step of realizing the narcosism is the adsorption onto the lipid film. Characteristics of the local anesthetics from the standpoint of the molecular structure consist in that they are an amphiphatic compound having a hydrophobic group comprising an aromatic ring and a hydrophilic group such as amino group. For comparison, partition coefficients to the lipid film and potency were determined for other amphiphatic substances such as sodium dodecanoate ($C_{11}COO^{-}$), dodecylamine hydrochloride ($C_{12}NH_3^{+}$), and naphthalene carboxylic acid dodecyl ester (Nap-$C_{12}$) as shown in Fig. 8. The results of $C_{11}COO^{-}$ and Nap-$C_{12}$ were far from the above linear correlation, but that of $C_{12}NH_3^{+}$ only was plotted near the above linear correlation. The results of the above experiments show that both hydrophobic group and amino group are necessary for the compound exhibiting local anesthetic activity, and such compounds as not to have amino group exhibit no anesthetic activity even if they are adsorbed onto the lipid film. As the result, it is considered that the molecular characteristics of medicinal substances having local narcosism consist in that they are amphiphatic compounds having amino group and hydrophobic group.

It is known that the local anesthetics have amino group in the molecule, and correlation between changes of Log P as changes of adsorption behavior and changes of pH in the aqueous phase was determined as shown in Fig. 9. In the case of tetracaine hydrochloride (TC) as a typical local anesthetics, the above results show that the partition coefficient are increased above pH 7 and adsorption onto the lipid film becomes easy. It may be caused by the fact that in the lower pH range not higher than pH 7, the amino group may be protonized to be hydrophilic, but in the higher pH range, it may be neutralized to be hydrophobic. The above phenomenon coincide very well with the fact that it is difficult for the local anesthetics to act on an inflamed acid tissue in the living body, whereas it becomes easier to act on an alkaline tissue.

Partition coefficients of tetracaine hydrochloride to the lipid film at temperatures of 10 to 60° C were determined under two conditions of pH 7 and pH 11 respectively and the results are shown in Fig. 10. The result sh-ws that the values of partition coefficient at pH 11 are larger than those at pH 7 all over the temperature range, and that they are increased at about 45° C or higher at both pH values. From the fact that the $2C_{18}N^{+}2C_1/PSS^{-}$ bilayer film has a crystal-liquid crystals phase transition temperature (Tc) at 45° C, it is considered that the increase of the partition coefficient for the local anesthetics at higher temperatures may be caused by the above phase transition. In other words, the above result shows that adsorption of the local anesthetics is made easy when the lipid film is in the state of liquid crystals. This result is of great interest in connection with the fact that the lipid of the cellular film in the living body is in the state of liquid crystals.

The effect of increasing the partition coefficient due to To was about 1.3 times at pH 7, whereas that at

pH 11 was about 2.5 times. It is considered that the reason why the increased effect at pH 11 is in that the local anesthetics may be of neutralized ones at pH 11 and become more hydrophobic than at pH 7, resulting in being deeply adsorbed onto the alkyl chain moiety of the lipid film to be greatly influenced by melting of the alkyl chain of the lipid film. The above results obtained by use of the lipid bilayer film-coated crystal oscillator makes it possible to provide novel teachings of changes of adsorbed moiety in the lipid film due to changes in characteristics of medicinal substances.

In comparison with the local anesthetics, it is known that the inhalational general anesthetics have no characteristic molecular structure and the hydrophobic organic compounds in general exhibit narcosism, and it is considered that their narcosism may non-specifically be realized only by being solubilized into the lipid film. On the other hand, it is known that the local anesthetics are an amphiphatic compound having amino group and phenyl group, and absence of the above structure results in exhibiting no anesthetic activity even if they have been adsorbed onto the lipid film. From the above facts, it is considered that the local anesthetics are adsorbed onto the lipid moiety of the synapse film of the nerve and are further received in the ion channel protein to realize their activity.

Example 4

As a method of evaluating toxicity of chemical substances to the living body, the Draize test, which is an animal experiment by use of the rabbit's eye, is known. According to the Draize test, a Draize score is determined for the chemical substances to evaluate their toxicity. Adsorption experiments of 70 chemical substances, Draize scores of which are known, were carried out by use of the same lipid bilayer film-coated crystal oscillator as in Example 2. Respective 10 wt% ethanol solutions of these chemical substances were prepared and were injected into distilled water at 45°C in a small amount respectively for carrying out the adsorption experiment. From the results of the adsorption experiment, respective partition coefficients (Log P) were determined. Respective Draize scores were plotted along the axis of ordinates, and partition coefficients (Log P) were plotted along the axis of abscissas to obtain a linear correlation as shown in Fig. 11 to show that the chemical substances, which are readily adsorbable in the lipid bilayer film, are liable to damage the rabbit's eye. Of the above 70 chemical substances, 34 chemical substances showing good linear correlation were such as to show a correlation coefficient ($\gamma$) of 0.872624. The names and structural formulas of the above 70 chemical substances are listed in the following Table-1 along with their numbers.

Table－1

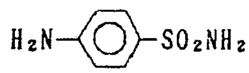

1. Suifanilamide

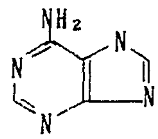

2. Adenine

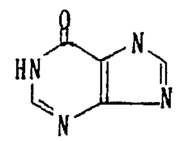

3. Hypoxantine

$H_2NCH_2COOH$

4. Glycine

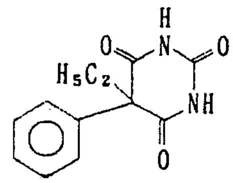

5. Phenobarbital

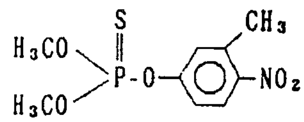

6. Fenitrothion(Sumithion)

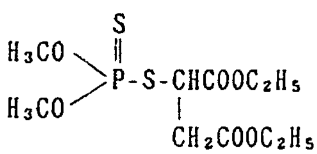

7. Malathion

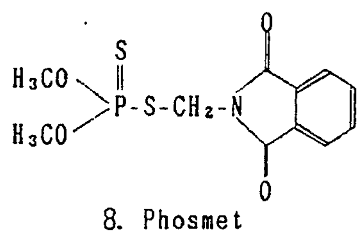

8. Phosmet

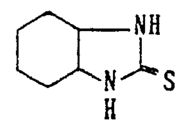

9. Octahydro-2H-benzimidnzole-2-thione

$CH_3CH_2SCH_2CH_2CH(NH_2)COOH$

10. Ethionine

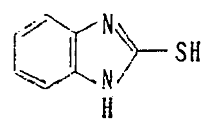

11. 2-Benzimidazolcthiol

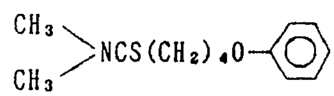

12. Fenothiocarb (Panocon)

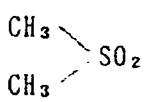

13. Dimethylsulfone

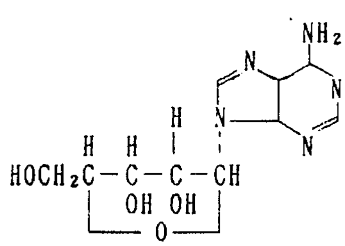

14. Adenosine

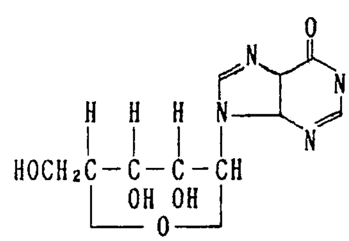

15. Inosinet

Table－1 (cont.)

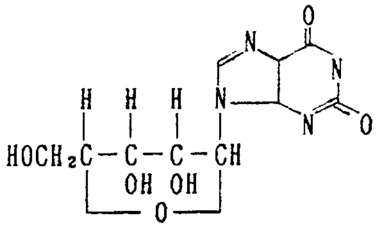

16. Guanoslne

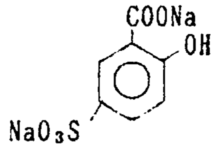

17. 5-Sulfosalicylic acid Na salt

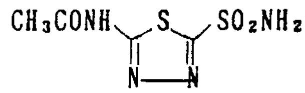

18. Acetazolamide

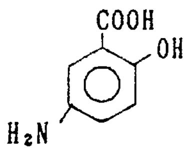

19. 5-Aminosalicylic acid

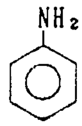

20. Aniline

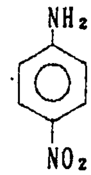

21. P-Nitroaniline

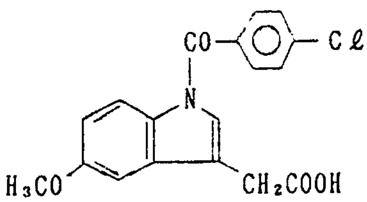

22. Indomethacin

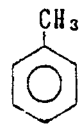

23. Toluene

$H_3COH$

24. Methyl alchol

$C_2H_5OH$

25. Ethyl alchol

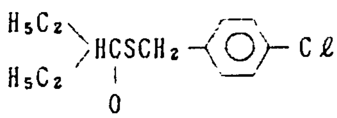

26. Benthiocarb(Saturn)

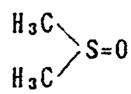

27. Dimethylsulfoxide

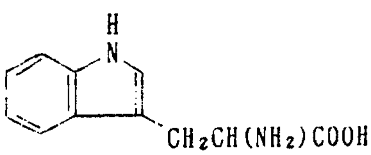

28. L-Tryptophane

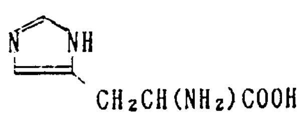

29. L-Histidine

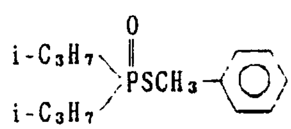

30. IBP(Kitazin-P)

Table-1 (cont.)

| | | |
|---|---|---|
| $H_3CCOCH_2COCH_3$ | 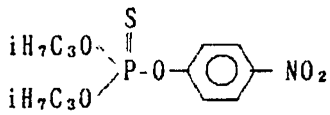 | 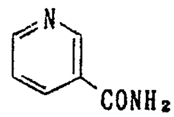 |
| 31. Acetylacetone | 32. O,O' -diisopropyl-O-(p-nitrophenylthiophosphate) | 33. Nicotineamide |
| 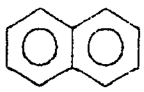 | 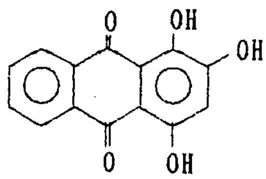 | 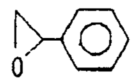 |
| 34. Naphtalene | 35. Purpurine | 36. 1,2-Epoxyethylbenzene |
| 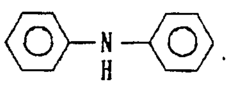 | 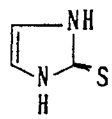 | $C_2H_5OC_2H_5$ |
| 37. Diphenylamine | 38. 2-Imidazolethion | 39. Diethylether |
| 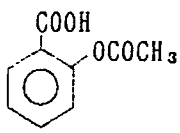 | 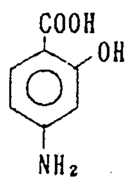 | 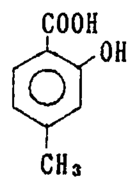 |
| 40. Acetylsallcylic acid | 41. 4-Aminosalicylic acid | 42. 4-Methylsalicylic acid |
| 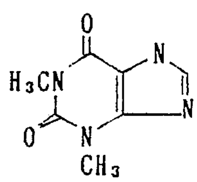 | 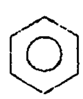 | 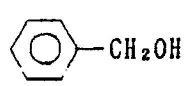 |
| 43. Caffein | 44. Benzene | 45. Benzyl alchol |

Table－1 (cont.)

$C_4H_9OH$

46. Buthyl alchol

$C_8H_{17}OH$

47. Octyl alchol

$H_3CCSNH$

48. Thloacetamide

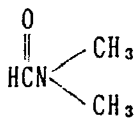

49. Dimethylformamid

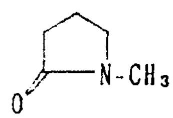

50. N-Methyl-2-pyrrolidone

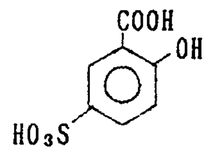

51. 5-Sulfosalicylie acid

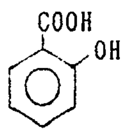

52. Salicylic acid

$C\ell CH_2SO_3C\ell$

53. Chloromethylsulfonyl chloride

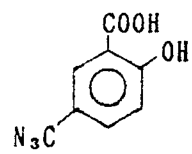

54. 5-Methylsalicylic acid

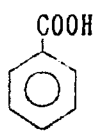

55. Benzoic acid

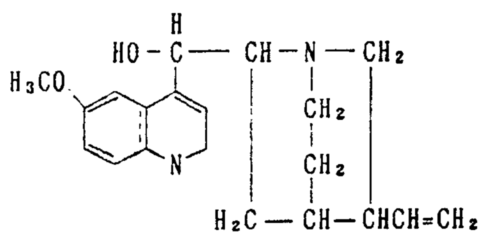

56. Qulnine

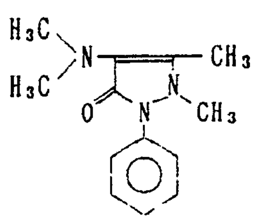

57. Dimethylaminoantipyrine

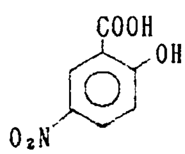

58. 5-Nitorosalicylic acid

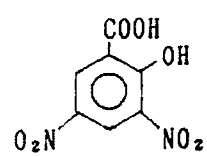

59. 3,5-Dinitroaalicylic acid

Table-1 (cont.)

$[H_3C(CH_2)_{10}CH_2SO]^-Na^+$

60. Sodium dodecylsulfate

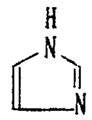

61. Imidazole

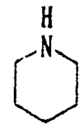

62. Piperidine

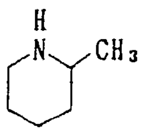

63. 2-Pipecoline

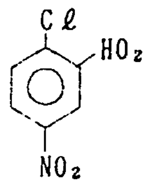

64. 2,4-Dinitrochloro benzene

$HSCH_2CH_2OH$

65. Mercaptoethanol

$CH_2SO_3H$

66. Methansulfonic acid

$C_2H_5SO_3H$

67. Ethansuifonic acid

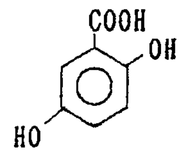

68. 5-Hydroxysalicyllc acid

$HS-CH_2CH(NH_2)COOH$

69. Cysteine

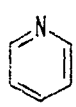

70. Piridine

As shown in the above Examples, the lipid bilayer film-coated crystal oscillator not only is an effective device to detect simple adsorption and interaction between the medicinal substances and the lipid film, but also may be a useful means to carry out studies on mechanisms of pharmacological activity.

## Claims

1. A method of evaluating the physiological activities and structural characteristics of medicinal substances, which method comprises determining correlations of (A) at least one of kinds of lipid films, physiological activity factors of medicinal substances and structural characteristics of medicinal substances with (B) at least one of amounts of change in frequency, adsorbed amounts or interacted amounts due to adsorption or interaction of the medicinal substances onto the lipid films, and lipid film-water partition coefficients, by use of crystal oscillators cast various lipid films respectively.

2. A method as claimed in claim 1, wherein the lipid film-water partition coefficient is used as a parameter in the structural activity correlation technique for medicinal substances.

3. A method as claimed in claim 1, wherein said correlation is such that the lipid film is $2C_{14}$ phosphoethanolamine based phospholipid film, the medicinal substrate is cyclic peptide based antibiotics, and it is determined from frequency changes, adsorbed or interacted amounts, penetration coefficient (D)

and partition coefficient (P) that the cyclic peptide based antibiotics are specifically adsorbed onto the phospholipid film and an interaction takes place therebetween so that the antibiotics penetrate into the interior of the phospholipid film to realize their physiological activities.

4. A method as claimed in claim 1, wherein said correlation is such that the lipid film is a polyion complex lipid film, the medicinal substance is a long chain fatty acid, and it is determined from adsorbed or interacted amounts measured from frequency changes and from partition coefficients of the long chain fatty acid to the lipid film that the antimicrobial activity against the Gram positive bacteria of the long chain fatty acid as the physiological activity of the medicinal substance has a good correlation with the partition coefficient of the long chain fatty acid to the lipid film so that the long chain fatty acid may be adsorbed onto the lipid film for realizing antimicrobial activity against the Gram positive bacteria.

5. A method as claimed in claim 1, wherein said correlation is such that the lipid film is a polyion complex lipid film, the medicinal substance is local anesthetics, and it is determined from the partition coefficient obtained from adsorbed or interacted amounts that the intrinsic potency as the physiological activity of the local anesthetics has a good linear correlation with the partition coefficient of the local anesthetics to the lipid film so that the local anesthetics are adsorbed onto the lipid film to realize their physiological activity.

6. A method as claimed in claim 1, wherein the lipid film is a polyion complex lipid film, the medicinal substances are chemical substances, and it is determined from the partition coefficients of the chemical substances to the lipid film, which are obtained from adsorbed amounts, that the Draize scores as an indication of the toxicity of chemical substances have a good correlation with the partition coefficients so that the chemical substances are adsorbed onto the lipid film to realize their toxicity.

FIG. 1

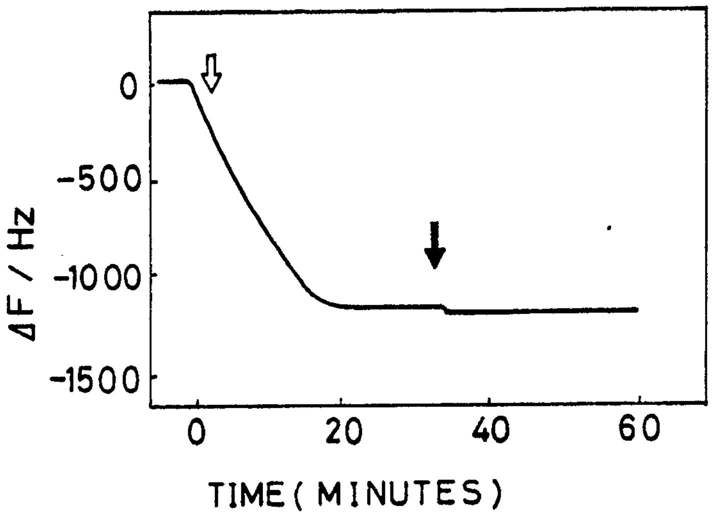
0
-500
-1000
-1500
ΔF / Hz
0
20
40
60
TIME ( MINUTES )

FIG. 2

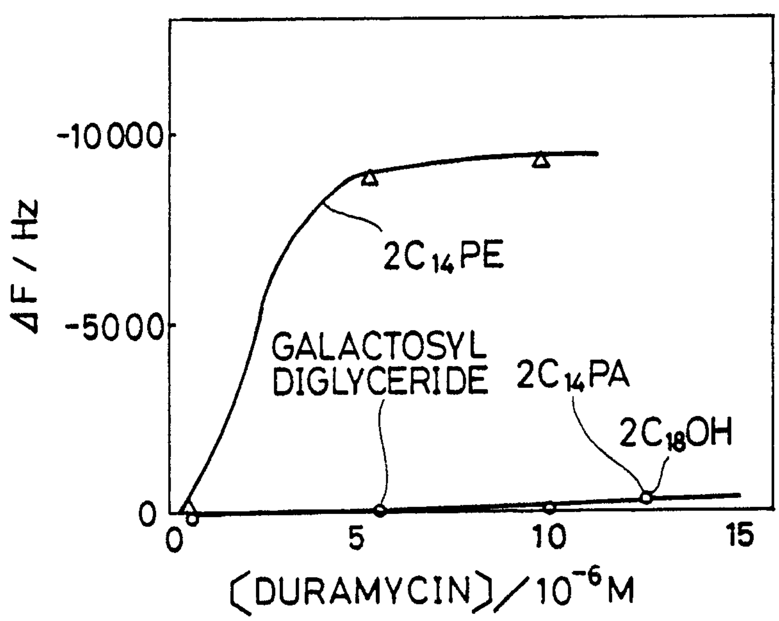
-10000
-5000
0
ΔF / Hz
2C14PE
GALACTOSYL
DIGLYCERIDE
2C14PA
2C18OH
0
5
10
15
〔DURAMYCIN〕/ 10-6M

FIG. 3

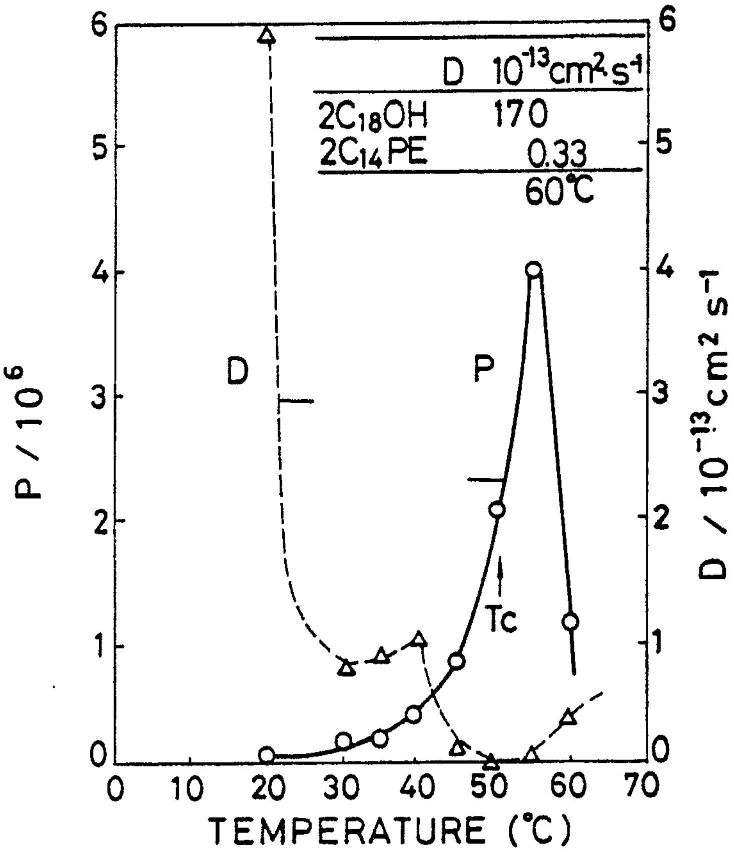
D 10^-13cm^2·s^-1
2C18OH 170
2C14PE 0.33
60℃
D
P
Tc
P / 10^6
D / 10^-13 cm^2 s^-1
0 10 20 30 40 50 60 70
TEMPERATURE (℃)

FIG. 4

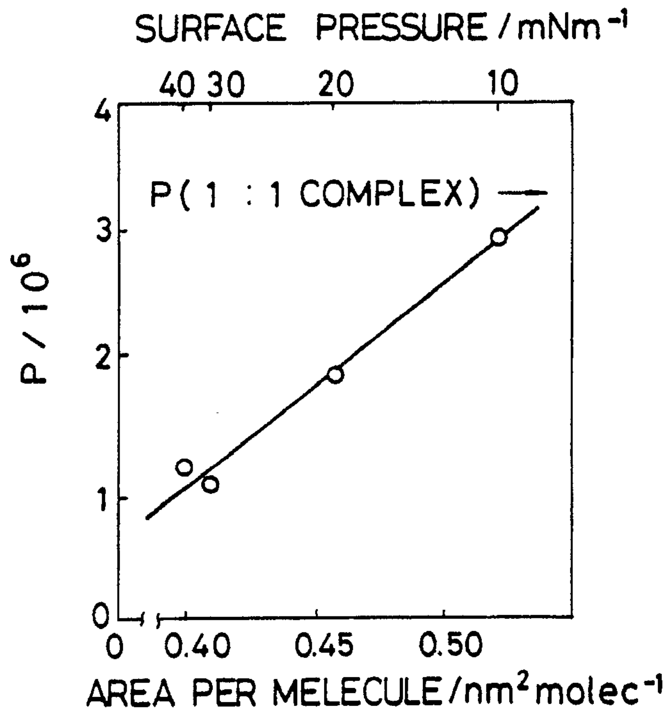
SURFACE PRESSURE / mNm^-1
40 30 20 10
P ( 1 : 1 COMPLEX)
P / 10^6
0 0.40 0.45 0.50
AREA PER MELECULE / nm^2 molec^-1

FIG. 5

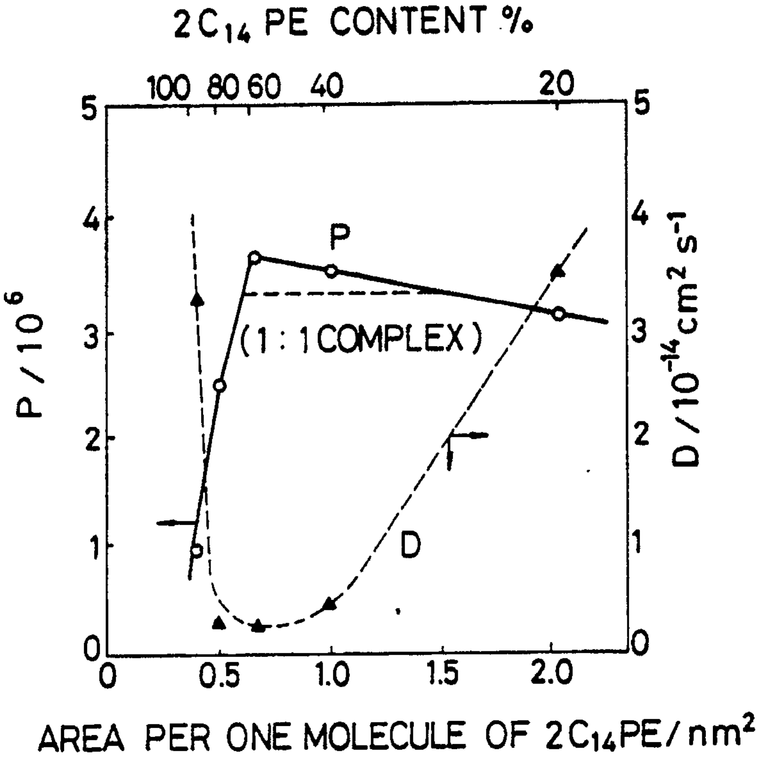
2C14 PE CONTENT %
100 80 60 40 20
P / 10^6
D / 10^-14 cm^2 s^-1
P
(1 : 1 COMPLEX)
D
0 0.5 1.0 1.5 2.0
AREA PER ONE MOLECULE OF 2C14PE/nm^2

FIG. 6

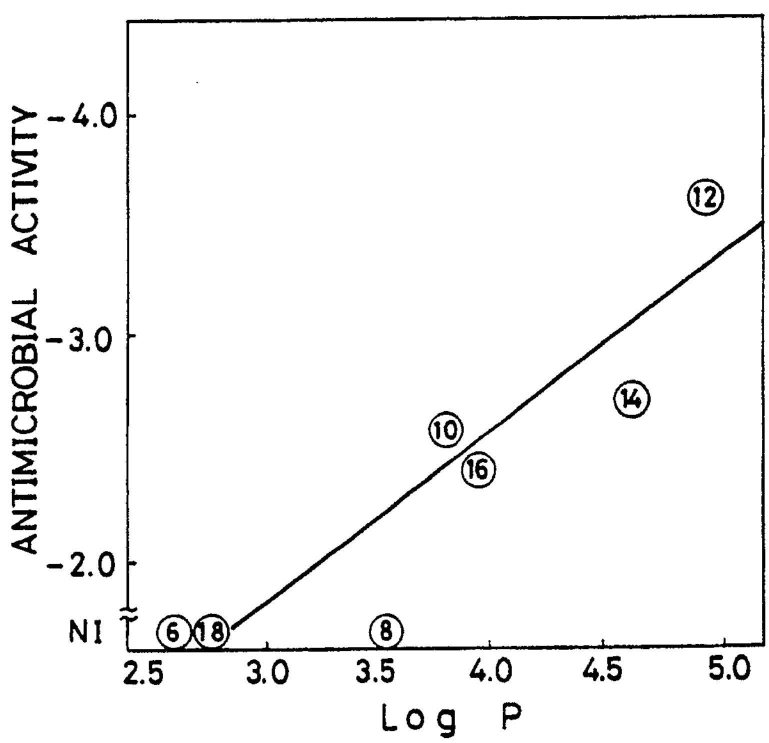
ANTIMICROBIAL ACTIVITY
-4.0
-3.0
-2.0
NI
12
14
10
16
6
18
8
2.5 3.0 3.5 4.0 4.5 5.0
Log P

FIG.7

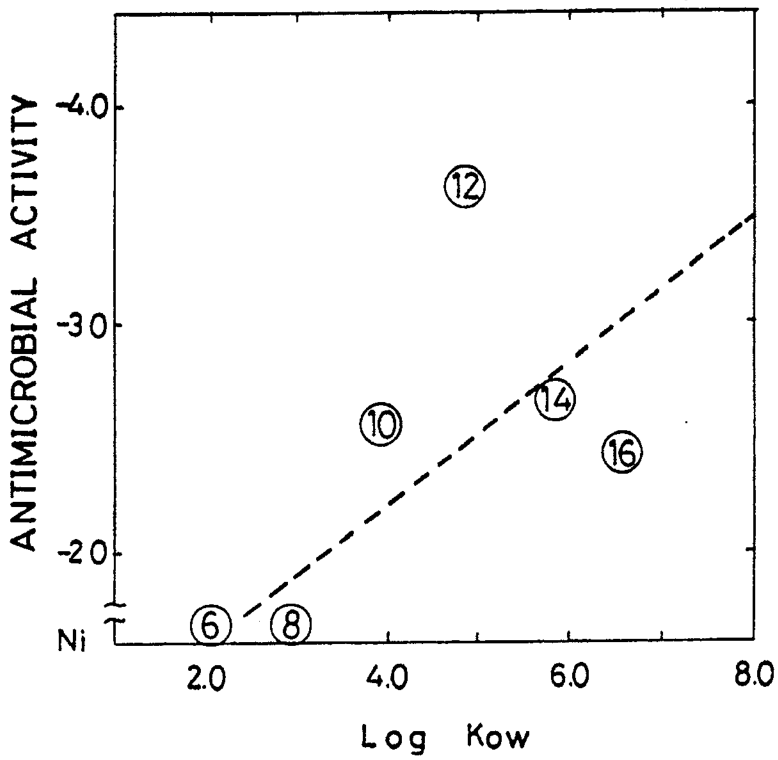
ANTIMICROBIAL ACTIVITY
-4.0
-3.0
-2.0
Ni
12
10
14
16
6
8
2.0
4.0
6.0
8.0
Log Kow

FIG.8

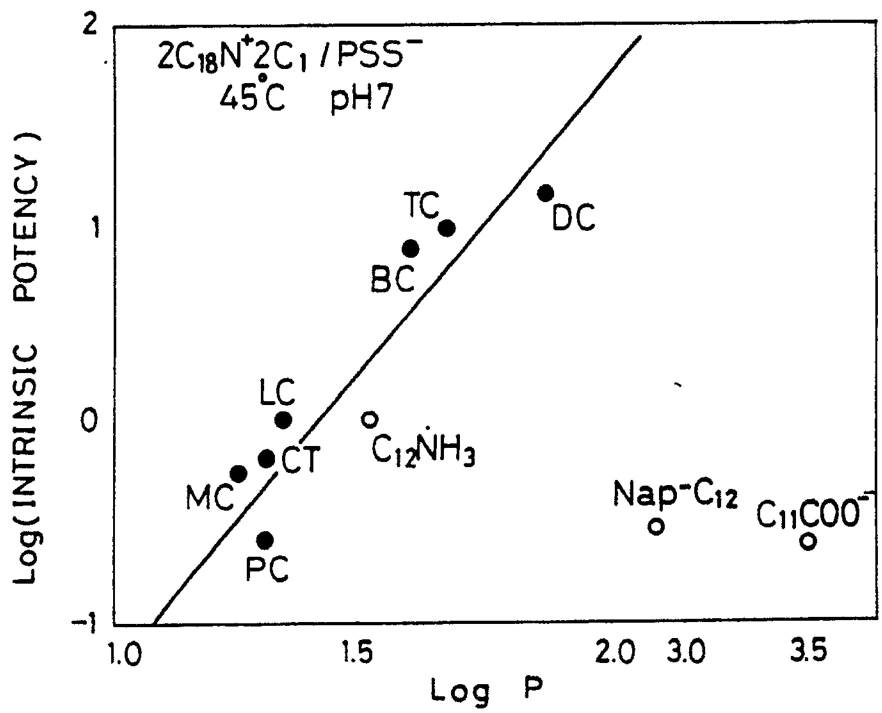
2C18N+2C1 / PSS−
45°C pH7
Log( INTRINSIC POTENCY )
2
1
0
−1
TC
DC
BC
LC
CT
MC
PC
C12NH3
Nap-C12
C11COO−
1.0
1.5
2.0
3.0
3.5
Log P

# FIG.9

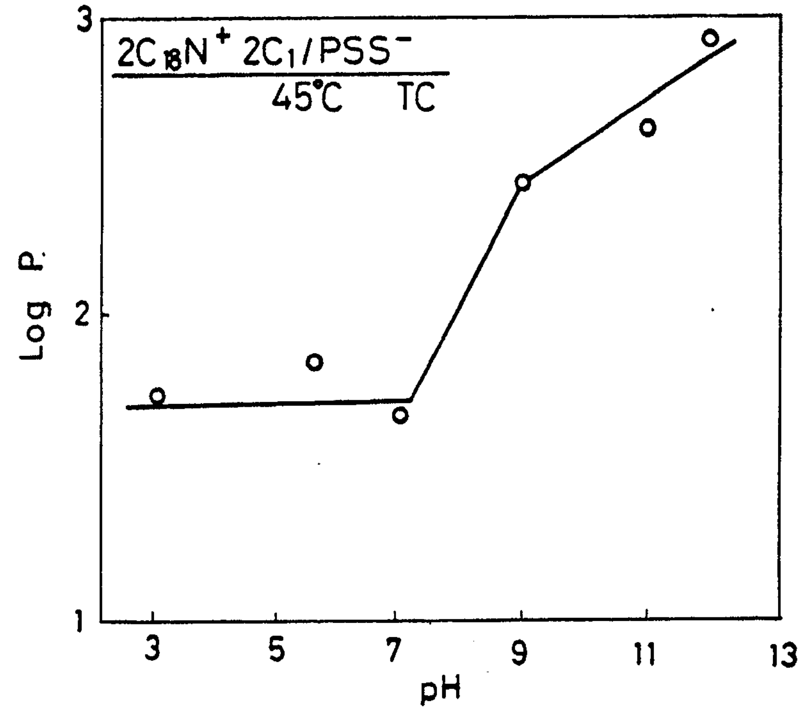

3
2
1
Log P
$2C_{18}N^+2C_1/PSS^-$
45°C TC
3
5
7
9
11
13
pH


# FIG.10

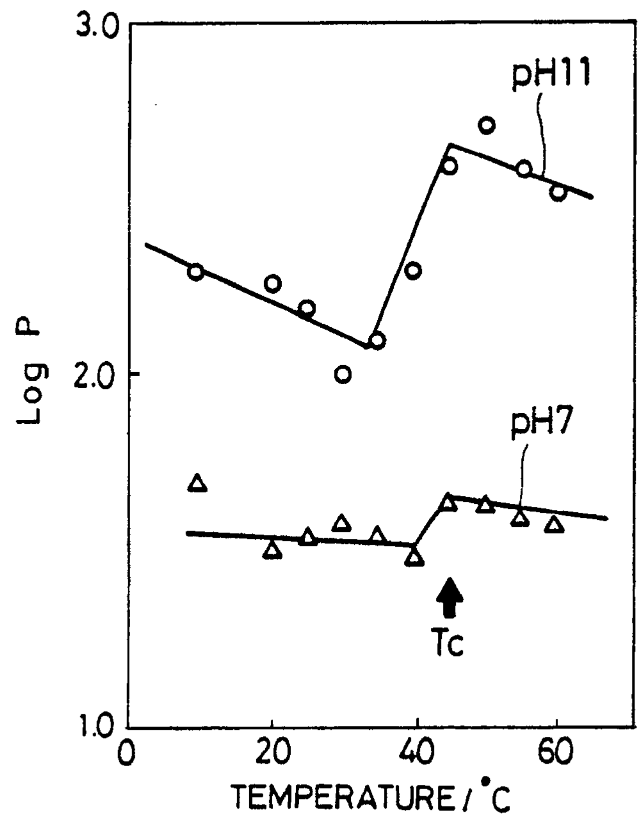

3.0
2.0
1.0
Log P
pH11
pH7
Tc
0
20
40
60
TEMPERATURE / °C

FIG. 11